# EUROPEAN PATENT SPECIFICATION

(11) **EP 0 674 617 B1**
(45) Date of publication and mention of the grant of the patent: **08.10.1997**
(21) Application number: 94903788.1
(22) Date of filing: 13.12.1993
(51) Int. Cl.: C07C 303/24, C07C 303/44, C11D 1/12

(54) **SECONDARY ALKYL SULPHATE/ZEOLITE-CONTAINING SURFACTANT COMPOSITION AND ITS OBTAINING PROCESS**
SEKUNDARALKYLSULFAT/ZEOLIT-HALTIGES TENSIDGEMISCH UND DESSEN HERSTELLUNGSVERFAHREN
COMPOSITION TENSIOACTIVE RENFERMANT UNE ZEOLITHE ET UN SULFATE D'ALKYLE SECONDAIRE ET SON PROCEDE D'OBTENTION

(30) Priority: 15.12.1992 US 990920
(43) Date of publication of application: 04.10.1995
(73) Proprietor: SHELL INTERNATIONALE RESEARCH MAATSCHAPPIJ B.V., 2596 HR Den Haag (NL)
(72) Inventor: SCHISLA, David, Karl, Houston, TX 77082 (US); TOMASKOVIC, Robert, Stephen, Houston, TX 77083 (US); LUTZ, Eugene, Frederick, Houston, TX 77042 (US); ARBORE, Charles, Michael, Houston, TX 77079 (US)
(86) International application number: PCT/EP93/03557
(87) International publication number: WO 94/13632

(56) References cited:
- EP-A- 0 466 243
- EP-A- 0 544 492
- WO-A-93/24452
- Derwent's abstract, no. 93-124137/15, week 9315; & SU,A,1707058, (GRUZGORNOKHIMPROM PRODN ASSOC), 20-09-1988

## Description

This invention relates to a process for preparing surfactant compositions comprising a secondary alkyl sulphate, and the surfactant compositions so prepared. More in particular, the invention relates to secondary alkyl sulphate/zeolite-containing compositions and to a process for preparing the compositions.

In EP-A-0 466 243 WO-A-9324452 solid secondary alkyl sulphate compositions and processes for their preparation are taught. Such compositions are compatible with conventional surfactant compositions which are substantially free of water and unreacted organic matter (UOM). Such compositions thus allow maximum handling flexibility.

However, to prepare surfactant compositions in the form of an anhydrous free flowing powder, the disclosed process teaches to dry the intermediate composition excessively (heating up to 106 °C). In addition, it is suggested to add sodium carbonate to the already recovered solid secondary alkyl sulphate-containing surfactant composition.

The inventors set out to provide a simplified process for preparing surfactant compositions in the form of an anhydrous free flowing powder wherein the excessive heating step is avoided and wherein the process yields free flowing powder without requiring subsequent mixing tanks.

A process for preparing surfactant compositions has been found in which secondary alkyl sulphates derived from olefins and/or alcohols can be generated in a manner such that the secondary alkyl sulphate/zeolite-containing product is a solid. The surfactant composition can be used as a surfactant and/or detergent composition in particular for household applications (free flowing powder).

The invention further provides solid surfactant compositions comprising a secondary alkyl sulphate and a zeolite which are substantially free of unreacted organic matter and water. These surfactant compositions are prepared by the process of claim 1.

Specifically, the present process comprises the steps of:
a) sulphating a (C₈ to C₂₂) olefin and/or a (C₈ to C₂₂) secondary alcohol, optionally containing a primary alcohol, with a sulphating agent,
b) neutralising the sulphation product comprising alkyl sulphuric acid and dialkyl sulphate produced in step a) with a base in aqueous solution,
c) heating the product of step b) to saponify the dialkyl sulphate and to remove substantially all of the water from the mixture,
d) cooling the product of step c) in the presence of a nonionic organic liquid diluent to ambient temperature whereby a crystallised solid composition separates, and
e) recovering and drying the crystallised solid composition of step d).
wherein the nonionic organic liquid diluent is added at some point prior to step d), characterized in that a zeolite is added in step c) and/or step d).

Surprisingly, as illustrated by the attached examples, the zeolite may be introduced into the composition in step c) and/or step d), whereby the extensive drying as taught in the international application (heating up to 106 °C) is avoided.

In the foregoing process, the nonionic organic liquid diluent can be added during the sulphation step, following the sulphation step, or during the neutralisation or saponification steps, provided the diluent is substantially inert in the particular step in which it is added. It is not critical at what point the nonionic organic liquid diluent is added as long as the diluent is present prior to crystallisation step.

As used herein, the phrase "substantially free of unreacted organic matter and water" refers to compositions which contain less than 10 percent by weight (%wt), preferably less than 5 %wt, of unreacted organic matter and less than 5 %wt, preferably less than 2 %wt, of water.

In step a) of the present process olefins, secondary alcohols or a mixture thereof are sulphated. In a preferred embodiment, the alkyl sulphuric acid of step a) is prepared by the sulphation of olefins or by the sulphation of a mixture of olefins and alcohols.

Suitable olefins, alcohols and sulphating agents are known to the skilled person and are for instance disclosed in WO-A-9324452 mentioned above. In particular olefins in the C₁₂ to C₁₈ range, and concentrated sulphuric acid as sulphating agent are considered suitable. Likewise, suitable non-ionic organic liquid diluents such as heptane, toluene, isooctane, nonane and mixtures thereof are known from this application. In addition, a suitable process for carrying out step a) up to and including step e) may be found in this document.

The zeolites suitable for use in the present invention are typically those zeolites which are suitable for use as builders in detergent compositions. As used herein, the term "zeolite" is used to refer to crystalline aluminosilicate minerals of a cage-network structure with pores a few 10⁻¹⁰ m (Angstroms) in diameter. Some of the common materials, such as zeolite Y (faujasite) or zeolite A have a three dimensional structure with pore intersections ("supercages") somewhat larger than the pore size. Others such as zeolite L and mordenite have channels. For each type of zeolite a theoretical crystal structure or "framework" can be specified which is composed of interconnected silicon atoms, aluminium atoms and oxygen atoms arranged in an ordered fashion. The aluminium found within the framework is referred to as "framework aluminium". A typical zeolitic framework comprises corner-sharing SiO4 and AlO4 tetrahedra. Excess negative charges in the Si-O-Al framework are balanced by the presence of suitable positive ions such as hydrogen, ammonium, alkali metal, alkaline earth metal, rare earth metal, etc. Each specific zeolite will have either a specific Si to Al ratio or specified range of Si to Al ratios that correspond to the theoretical crystal structure of such zeolite type.

Zeolites which are suitable for use in the present invention are zeolite A, zeolite X, zeolite Y and mixtures thereof, with zeolite A being particularly preferred. Zeolite A is commercially available from sources such as The PQ Corporation under the trademark VALFOR 100, and the Ethyl Corporation. The amount of zeolite used in the present process is typically an amount such that the final secondary alkyl sulphate/zeolite composition contains at least 25 %wt, preferably from 50 to 75 %wt (based on the total weight of the composition).

The zeolite may be added during the saponification step (step c), or during the crystallisation step (step d). The zeolite is believed to act as a filter aid in the recovery of the secondary alkyl sulphate/zeolite-containing product. It is also within the scope of the present invention to add the zeolite in a combination of steps such as, for example, the zeolite can be added prior to crystallisation, during crystallisation and following crystallisation, but before recovery.

Following saponification and removal of substantially all of the water, the resulting product is then cooled, preferably to a temperature in the range of from 20 °C to 85 °C, in the presence of the non-ionic organic liquid diluent to effect crystallisation of the solid secondary alkyl sulphate from the saponified product of step c). The cooling step generally takes place over a period of 0.25 hours to 18 hours, preferably 0.5 hours to 16 hours, although both shorter and longer time periods are also acceptable. During the cooling step, the solubility of secondary alkyl sulphate is further reduced and additional secondary alkyl sulphate solids are formed.

The crystallised secondary alkyl sulphate/zeolite product is then recovered and dried as solid secondary alkyl sulphate/zeolite-containing composition. The secondary alkyl sulphate/zeolite crystals can be recovered by filtration or centrifugation. The crystallised secondary alkyl sulphate/zeolite-containing product is typically dried at temperatures in the range of from 40 °C to 80 °C using nitrogen-swept vacuum or other conventional drying means. Prior to drying, the crystals may be subjected to washing with the particular non-ionic organic liquid diluent utilised or with any other conventional washing agent in order to increase the purity of the secondary alkyl sulphate/zeolite-containing product.

The remainder of the product of step d) which contains unreacted starting material, secondary alcohol, and non-ionic organic liquid diluent, as well as some uncrystallised secondary alkyl sulphate, may be recycled. Alternatively, the uncrystallised secondary alkyl sulphate may be removed from the product of step d), if desired, prior to recycle. In addition, if desired, the secondary alcohol can be separated from the unreacted starting material by means recognised by those skilled in the art such as, for example, distillation.

The process of the present invention may be carried out in a batch mode or in a continuous operation.

The solid secondary alkyl sulphate/zeolite-containing surfactant compositions prepared in the present process typically contain at least 80 %wt (based on the total weight of the composition) of a combination of secondary alkyl sulphate and zeolite, and preferably at least 90 %wt of a combination of secondary alkyl sulphate and zeolite. The solid secondary alkyl sulphate/zeolite product produced contains preferably 25 to 75 %wt, more preferably 25 to 50 %wt of secondary alkyl sulphate, and preferably 25 to 75 %wt, more preferably 50 to 75 %wt of zeolite. The product generally contains some residual level of sodium sulphate. The product typically contains less than 20 %wt, preferably less than 12 %wt, more preferably less than 9 %wt (based on the total weight of the composition) of sodium sulphate.

The invention will be described below by the following examples which are provided for purposes of illustration and are not to be construed as limiting the invention.

### Illustrative Embodiments

### Example 1

To a three necked 2-litre jacketed glass vessel with a paddle stirrer, thermometer, and addition flask topped with a nitrogen blanket was added 400 grams of 97 %wt C16 linear alpha olefin. Glycol chilled to 0 °C was circulated through the vessel jacket. 115 Grams of 95 %wt sulphuric acid were added with good agitation at a rate such that the temperature did not exceed 20 °C. When the addition of sulphuric acid was complete, the mixture was agitated for about 1 hour. 200 Grams of heptane were then added to the mixture. 500 Grams of this mixture were then added to 212.5 grams of 20 %wt NaOH in a second three necked 2-litre glass vessel equipped with a paddle stirrer, thermometer, a Dean-Stark trap with a nitrogen blanket, and an additional flask. The temperature in the reaction flask rose from about 24 °C to 70 °C during acid neutralisation. When the acid addition was complete, 150 grams of heptane was added to this mixture.

The addition flask was replaced with a nitrogen blanketed Dean Stark trap and the reactor was heated to azeotropically distil the water from the reaction mixture. After 172 grams of water were removed and the reaction temperature reached 100 °C, 50 grams of the zeolite VALFOR 100 (VALFOR is a trademark of and sold by The PQ Corporation) were added to the reaction mixture. An additional 50 grams of heptane were also added to the reaction mixture. After azeotropically distilling another 16 grams of water, for a total of 188 grams, heating was discontinued and the heptane in the Dean Stark trap was returned to the reactor.

On cooling overnight to room temperature, the reactor contents were filtered using a 10 centimetre diameter Buchner funnel. The solid isolated on the filter was washed with 1200 grams of heptane. The solid product was then transferred to a tarred dish and dried at 60 °C and in a nitrogen swept vacuum of 74 x 10³ N/m² (22 inches of mercury) to a constant weight. The dried solid product weighed 193.8 grams. Analysis showed the recovered product to be 75.0 %wt anionic surfactant and 0.18 %wt sodium sulphate. An analysis was not performed on the zeolite but all that was added was retained on the filter and hence in the solid product, which accounts for the remaining 25 %wt of the product. The anionic surfactant yield was 35 % (mole/mole on the starting olefin). The results are presented in Table I.

### Example 2

Example 2 was carried out in a manner similar to Example 1, except that 150 grams of VALFOR 100 (VALFOR is a trademark) were added instead of 50 grams. 288 Grams of the solid product were recovered, containing 53.8 %wt anionic surfactant, 0.25 %wt sodium sulphate, and about 50 %wt zeolite. The anionic surfactant yield was 36 % (mole/mole). The results are presented in Table I.

### Example 3

Example 3 was carried out in a manner similar to Examples 1 and 2, except that the zeolite was added after the reaction mixture, or slurry, was cooled to room temperature. The process leading up to zeolite addition makes a solid product containing primarily anionic surfactant, with smaller amounts of sodium sulphate and sodium hydroxide. In Example 3, 150 grams of heptane were added after neutralisation instead of the 200 grams added in Example 1. 640 Grams of slurry were recovered. 214 Grams of the slurry were filtered without the addition of zeolite and washed with 404 grams of heptane to obtain 50.4 grams of a solid product containing 97.0 %wt anionic surfactant and 0.34 %wt sodium sulphate. 220 Grams of the slurry were blended with 100 grams of zeolite and this mixture was filtered and washed with 409 grams of heptane. 150 Grams of solid product were recovered which was 34.4 %wt anionic surfactant and about 66 %wt zeolite. The anionic surfactant yield on starting olefin for both cases was 36 % (mole/mole). Also, the filtration rates were not different, although the weight of the cake was about three times larger and the thickness of the cake was twice as large with the zeolite. The results are presented in Table I.

### Example 4

Example 4 was carried out in a similar manner to Example 3. Part of the slurry was blended with zeolite to give a solid product containing 35.5 %wt anionic surfactant with the remaining balance primarily zeolite. The slurry was also filtered without adding zeolite to give a solid product containing 99.1 %wt anionic surfactant and 1.3 %wt sodium sulphate. The anionic surfactant yield on olefin was again the same for both cases at 38 % (mole/mole). However, in Example 4 the filtration rate was three times faster for the slurry containing zeolite. The results are presented in Table I.

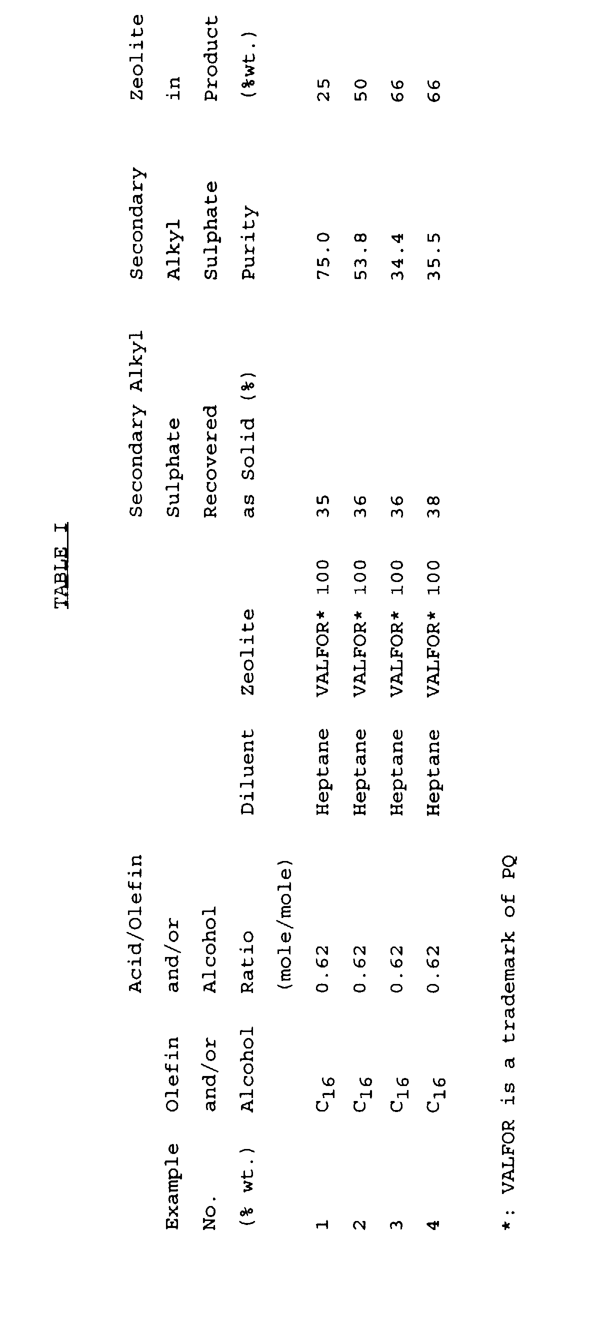

## Claims

1. A process for preparing a surfactant composition comprising a secondary (C₈ to C₂₂) alkyl sulphate, which process comprises:
a) sulphating a (C₈ to C₂₂) olefin and/or a (C₈ to C₂₂) secondary alcohol, optionally containing a primary alcohol, with a sulphating agent,
b) neutralising the sulphation product comprising alkyl sulphuric acid and dialkyl sulphate produced in step a) with a base in aqueous solution,
c) heating the product of step b) to saponify the dialkyl sulphate and to remove substantially all of the water from the mixture,
d) cooling the product of step c) in the presence of a non-ionic organic liquid diluent to ambient temperature whereby a crystallised solid composition separates, and
e) recovering and drying the crystallised solid composition of step d),
wherein the non-ionic organic liquid diluent is added at a point prior to step d), characterized in that a zeolite is added in step c) and/or step d).

2. A process as claimed in claim 1, wherein the zeolite is selected from the group consisting of zeolite A, zeolite X, zeolite Y and mixtures thereof.

3. A process as claimed in 2, wherein the zeolite is zeolite A.

4. A process as claimed in claim 1, 2 or 3, wherein the solid secondary alkyl sulphate-containing surfactant composition recovered in step e) contains from 25 to 75 %wt secondary alkyl sulphate and from 75 to 25 %wt of zeolite.

5. A surfactant composition that is solid at ambient temperature, prepared according to claim 1, characterized in that it comprises at least 80 %wt of a combination of a secondary (C₈ to C₂₂) alkyl sulphate and zeolite; less than 20 %wt of sodium sulphate; less than 10 %wt of the corresponding (C₈ to C₂₂) olefin and/or (C₈ to C₂₂) alcohol, and less than 5 %wt of water.

## Patentansprüche

1. Ein Verfahren zur Herstellung eines ein sekundäres (C₈-C₂₂) Alkylsulfat umfassenden Tensidgemisches, welches Verfahren umfaßt:
a) Sulfatieren eines (C₈-C₂₂)-Olefins und/oder eines (C₈-C₂₂)sekundären Alkohols, gegebenenfalls mit einem Gehalt an einem primären Alkohol, mit einem Sulfatierungsmittel,
b) Neutralisieren des in Stufe a) ausgebildeten Sulfatierungsproduktes, das Alkylschwefelsäure und Dialkylsulfat umfaßt, mit einer Base in wässriger Lösung,
c) Erwärmen des Produktes aus Stufe b) zum Verseifen des Dialkylsulfats und zur Abtrennung von im wesentlichem dem gesamten Wasser aus dem Gemisch,
d) Abkühlen des Produktes aus Stufe c) in Anwesenheit eines nichtionischen organischen flüssigen Verdünnungsmittels auf Umgebungstemperatur, wobei sich eine kristallisierte feste Zusammensetzung ausscheidet und
e) Gewinnen und Trocknen der kristallisierten festen Zusammensetzung aus Stufe d),worin das nichtionische organische flüssige Verdünnungsmittel in einem Stadium vor der Stufe d) zugesetzt wird, dadurch gekennzeichnet, daß in Stufe c) und/oder d) ein Zeolith zugesetzt wird.

2. Verfahren nach Anspruch 1, worin der Zeolith aus der aus Zeolith A, Zeolith X, Zeolith Y und Gemischen hievon bestehenden Gruppe ausgewählt ist.

3. Verfahren nach Anspruch 2, worin der Zeolith Zeolith A ist.

4. Verfahren nach Anspruch 1, 2 oder 3, worin die in Stufe e) gewonnene feste, sekundäres Alkylsulfat enthaltende Tensidzusammensetzung von 25-75 Gew.-% sekundäres Alkylsulfat und von 75-25 Gew.-% Zeolith enthält.

5. Bei Umgebungstemperatur festes Tensidgemisch, hergestellt nach Anspruch 1, dadurch gekennzeichnet, daß es wenigstens 80 Gew.-% einer Kombination aus einem sekundären (C₈-C₂₂) Alkylsulfat und Zeolith; weniger als 20 Gew.-% Natriumsulfat; weniger als 10 Gew.-% des entsprechenden (C₈-C₂₂)Olefins und/oder (C₈-C₂₂) Alkohols und weniger als 5 Gew.-% Wasser umfaßt.

## Revendications

1. Procédé de préparation d'une composition de tensioactif ou de surfactif comprenant un alkyl(C₈ à C₂₂)sulfate secondaire, caractérisé en ce qu'il comprend les étapes consistant à :
a) sulfater une oléfine (C₈ à C₂₂) et/ou un alcool secondaire (C₈ à C₂₂), contenant éventuellement un alcool primaire, avec un agent de sulfatation,
b) neutraliser le produit de la sulfatation, qui comprend de l'acide alkylsulfurique et du dialkylsulfate, produit au cours de l'étape a), avec une base en solution aqueuse,
c) chauffer le produit de l'étape b), de manière à saponifier le dialkylsulfate et à éliminer sensiblement la totalité de l'eau du mélange,
d) refroidir le produit de l'étape c), en présence d'un diluant liquide organique non ionique, jusqu'à la température ambiante, de manière à ce qu'une composition solide cristallisée se sépare et
e) récupérer et sécher la composition solide cristallisée de l'étape d),
où on ajoute le diluant liquide organique non ionique en un certain point préalablement à l'étape d), caractérisé en ce que l'on ajoute une zéolite, au cours de l'étape c) et/ou de l'étape d).

2. Procédé suivant la revendication 1, caractérisé en ce que l'on choisit la zéolite dans le groupe constitué par la zéolite A, la zéolite X, la zéolite Y et leurs mélanges.

3. Procédé suivant la revendication 2, caractérisé en ce que la zéolite est la zéolite A.

4. Procédé suivant l'une quelconque des revendications 1, 2 ou 3, caractérisé en ce que la composition de tensioactif ou surfactif contenant de l'alkylsulfate secondaire solide, récupérée dans l'étape e), contient de 25 à 75% en poids d'alkylsulfate secondaire et de 75 à 25% en poids de zéolite.

5. Composition surfactive ou tensioactive, qui est solide à la température ambiante, préparée suivant la revendication 1, caractérisée en ce qu'elle comprend au moins 80% en poids d'une combinaison d'un alkyl(C₈ à C₂₂)sulfate secondaire et une zéolite, moins de 20% en poids de sulfate de sodium, moins de 10% en poids de l'oléfine (C₈ à C₂₂) et/ou de l'alcool (C₈ à C₂₂) correspondant et moins de 5% en poids d'eau.
